# EUROPÄISCHE PATENTSCHRIFT

(11) **EP 2 029 759 B1**
(45) Veröffentlichungstag und Bekanntmachung des Hinweises auf die Patenterteilung: **14.10.2009**
(21) Anmeldenummer: 07729179.7
(22) Anmeldetag: 16.05.2007
(51) Int. Cl.: C12P 7/62, C08G 63/89

(54) **VERFAHREN ZUR ABTRENNUNG VON FERMENTATIV GEWONNENEN POLYHYDROXYALKANOATPARTIKELN UNTER VERWENDUNG EINES DÜSEN-SEPARATORS**
PROCESS FOR THE SEPARATION OF POLYHYDROXYALKANOATE PARTICLES OBTAINED BY FERMENTATION USING A NOZZLE SEPARATOR
PROCÉDÉ DE SÉPARATION DE PARTICULES DE POLYHYDROXYALCANOATES OBTENUES PAR FERMENTATION EN UTILISANT UN SÉPARATEUR À BUSES

(30) Priorität: 24.05.2006 EP 06114448
(43) Veröffentlichungstag der Anmeldung: 04.03.2009
(73) Patentinhaber: BASF SE, 67056 Ludwigshafen (DE)
(72) Erfinder: COOPER, Bryan, 68199 Mannheim (DE); SCHNELLER, Arnold, 64342 Seeheim-Jugenheim (DE); PREISHUBER-PFLÜGL, Peter, 67061 Ludwigshafen (DE)
(86) Internationale Anmeldenummer: PCT/EP2007/054731
(87) Internationale Veröffentlichungsnummer: WO 2007/135039

(56) Entgegenhaltungen:
- DATABASE WPI Week 199515 Derwent Publications Ltd., London, GB; AN 1995-109542 XP002448240 & JP 07 031488 A (ASAHI KASEI KOGYO KK) 3. Februar 1995 (1995-02-03)
- HARRISON, S.T.L. ET AL.: "The disruption of Alcaligenes eutrophus by high pressure homogenisation: Key factors involved in the process" BIOSEPARATION, Bd. 2, Nr. 3, 1991, Seiten 155-166, XP008082789 in der Anmeldung erwähnt

## Beschreibung

Die Erfindung betrifft ein Verfahren zur Isolierung von Polyhydroxyalkanoaten aus einer Produktionszelle.

Polyhydroxyalkanoate (PHA) wie z.B. Polyhydroxybutyrate (PHB) können mit Hilfe von Bakterien synthetisiert werden. Beispielsweise sind in Biopolymer, Wiley-VCH, 2002 derartige biotechnologische Verfahren beschrieben.

Das PHB liegt am Ende der Fermentation in den Bakterienzellen in Form von Körnchen vor, die von einer Proteinhülle umgeben sind (J. Biol. Chem. 1989, Vol. 264(6), Seiten 3286 - 3291). Um ein ausreichend reines PHB zu erhalten, muss dieses von den Bakterienzellen getrennt werden.

Die biotechnologisch hergestellten Rohmassen enthalten neben dem gewünschten Polyhydroxyalkanoat die Mikroorganismen, die das Polyhydroxyalkanoat produziert haben (Produktionszellen, Biomasse oder nicht-Polyhydroxyalkanoat-Masse). Die Isolierung des Polyhydroxyalkanoats aus der Biomasse kann a) durch Lösen der Biomasse, b) durch Extraktion des Polyhydroxyalkanoats in einem geeigneten Extraktionsmittel oder c) durch mechanischen Aufschluss der Biomasse (Produktionszelle) und anschließender Trennung der Zellfragmente von den Polyhydroxyalkanoat (PHA)-Körnchen erreicht werden.

Die häufigste Methode hierzu besteht in der Extraktion der PHA-Körnchen aus der Biomasse mit einem Lösungsmittel. Als geeignete Extraktionsmittel für Polyhydroxyalkanoate können chlorierte Verbindungen eingesetzt werden (Methode b)). Beispiele sind in EP 0124309 und der dort zitierten Literatur angeführt. Die Verwendung von Lösungsmitteln hat eine Reihe von Nachteilen zur Folge. Man ist gezwungen, in eine aufwendige und kostspielige Infrastruktur für die Handhabung und Rückgewinnung der Lösungsmittel zu investieren. Die extrahierte Biomasse muss vor der Weiterverwendung als Dünge- oder Futtermittel von den Lösungsmittelresten befreit werden. Da PHB sich nur ungenügend in vielen Lösungsmitteln löst, sind die Mengen an Lösungsmittel, die benötigt werden, sehr groß.

Zum Abbau und Lösen der Biomasse (Aufarbeitung a)) können beispielsweise Enzyme oder chemische Verfahren verwendet werden. Zusätzlich können oberflächenaktive Verbindungen beigefügt werden. Eine Kombination mehrere Verfahren ist möglich.

In EP 0145233 wird der Abbau der Biomasse durch Enzyme beschrieben.

In WO 94/24302 wird der Abbau der Biomasse durch Enzyme und Wasserstoffperoxid beschrieben.

In US 5110980 wird der Abbau der Biomasse durch Hypochlorit beschrieben, welcher Polyhydroxyalkanoaten mit hohem Molekulargewicht zugänglich macht. Unterschiedliche Parameter wie Temperatur, Zeit oder pH während der Behandlung mit Hypochlorit werden untersucht. Eine Reinigung des Polyhydroxyalkanoats mit verdünnten Säuren wird nicht beschrieben.

Eine weitere Methode zur Freisetzung des gebildeten Polyhydroxybutyrats (PHB) aus gentechnisch modifizierten Escherichia coli Zellen ist beschrieben worden (Research In Microbiology 2005, 156, Seiten 865-873). Hier wird eine Autolyse nachgeschaltet. Die genauen Autolysenbedingungen sind nicht beschrieben. Autolyse ist ein Prozess der Selbstauflösung der Zellen durch eigene Enzyme. Diese Herstellmethode hat folgende Nachteile. Da die Autolyse unvollständig verläuft und Zellfragmente sowie PHB-Körnchen noch aneinander haften, werden nur ca. 80% des gebildeten PHB' freigesetzt.

Die Patenschrift JP 7031488 A offenbart ein Verfahren zur Abtrennung von fermentativ gewonnenen Polyhydroxyalkanoatpartikeln durch einen Schritt der Zentrifugation, der sich dem Zellaufschluß durch Hochdruckhomogenisierung anschließt.

Die Aufgabe bestand daher darin, ein Verfahren zu finden, dass zu einer vollständigen Abtrennung der Zellfragmente der Produktionszelle von den gebildeten Polyhydroxyalkanoat-Körnchen führt.

Nachdem Versuche mit konventionellen Zentrifugen fehlgeschlagen waren, wurde überraschenderweise gefunden, dass die Zellfragmente von den Polyhydroxyalkanoat-Körnchen mittels eines kontinuierlich arbeitenden Düsenseparators sehr effizient getrennt werden können. Der Pellet bestehend aus den Polyhydroxyalkanoat-Körnchen wird durch die Düsen ständig abgeführt, während die Zellfragmente ständig im Überlauf (eigentlicher "Klarlauf") effektiv abgetrennt werden. Es gibt keine Entleerung durch Öffnen der Trommel und dadurch auch keine Verluste, Verwirbelungen und ähnliche Instabilitäten, die zu Lasten einer effizienten Trennung gehen. Um eine noch höhere Reinheit zu erlangen, können die PHA-Körnchen mit klarem Wasser versetzt und erneut zentrifugiert werden. Auf diese Weise gelangt man zu einer wässrigen Suspension von PHA-Körnchen hoher Reinheit, welche dann in bekannter Weise getrocknet werden kann, zum Beispiel durch Sprühtrocknung. Das entstehende Produkt ist geeignet für die Weiterverarbeitung zu thermoplastischen Kunststoffen. Die Verwendung von Lösungsmitteln ist nicht notwendig.

Das Verfahren zeichnet sich folglich gegenüber den herkömmlichen Verfahren durch eine hohe Effizienz, Wirtschaftlichkeit und eine exzellente Prozessfähigkeit aus.

Düsenseparatoren sind auch bekannt unter dem Begriff Westfalia Separator. Eine detaillierte Beschreibung ist beispielsweise unter www.gea-westfalia.de zu erhalten. Bei-spielhaft sei das VisCon®-System angeführt, bei dem die Düsen viskositätsgesteuert sind. Dadurch entfällt das Anpassen der Separatorparameter (Entleerungszeiten) bei veränderten Zulaufbedingungen und als Folge hiervon werden gleich bleibende Feststoffaustragskonzentrationen erreicht. Beim VisCon®-System liegen die Düsen nicht am Trommelrand, sondern auf einem kleineren Durchmesser in der Trommel. Die Einleitung über den hydrohermetischen Zulauf sowie der Ablauf über die Düsen erhöhen die Zellaktivität der abgetrennten Zellen.

Die notwendigen Apparate sind technisch verfügbar und beliebig upscale-fähig, so dass das Verfahren problemlos in den industriellen Maßstab übertragen werden kann.

Eine besondere Ausführungsform des erfindungsgemäßen Verfahrens schließt im Schritt i) die Produktionszellen mechanisch auf. Der chemikalienfreie Aufschluss hat Vorteile. Es ist beschrieben worden, dass man Zellen des PHB-produzierenden Bakteriums Alcaligenes eutrophus mit Hilfe eines Homogenisators aufschließen kann (Bioseparation 1991, 2, Seiten 155-166). Die in den Zellen vorhandenen PHB-Körnchen konnten nach viermaliger Passage durch einen Homogenisator fast vollständig aus den Zellen herausgelöst werden. Die Zellsuspension wird bei diesem Homogenisatortyp durch ein Ventil gepresst. Durch Verstellung des Spaltenabstandes zwischen Ventilkegel und Ventilsitz wird Turbulenz erzeugt. Die aus dem Ventil austretende Suspension prallt dann auf eine Stahlplatte. Der Druck dieser Maschine ist deshalb auf 1500 bar begrenzt.

Zur Erzeugung hoher Drücke werden großen Mengen elektrischen Stroms benötigt. Ein Zellaufschluss mit einem Homogenisator ist insbesondere dann wirtschaftlich, wenn die Zellen nach einer einzigen Passage vollständig aufgeschlossen werden. Die in Bioseparation 1991, 2, Seiten 155-166 beschriebene Methode hat den Nachteil, dass vier Passagen durch den Homogenisator benötigt werden.

Wir haben nun überraschenderweise festgestellt, dass PHA-haltige Zellen von insbesondere Alcaligenes eutrophus mit einem Hochdruckhomogenisator, wie er im folgenden beschrieben ist, sich sehr gut aufschließen lässt. Dabei werden bei einmaliger Passage durch den Hochdruckhomogenisator bei einem Druck von 2000 und mehr bar über 99% der Zellen aufgeschlossen und PHA nahezu vollständig freigesetzt. Gegen-Stand der vorliegenden Erfindung ist daher auch der Aufschluss mittels eines Hochdruckhomogenisators, der mit Drucken von 2000 und mehr atm arbeitet. Beispielsweise besteht er aus folgender Anordnung:

### Beispiele für geeignete Homogenisiervorrichtungen:

a) aus einer Blende mit wenigstens einer Eintrittsdüse und einer Blende mit wenigstens einer Austrittsdüse besteht, wobei im Zwischenraum zwischen den Blenden gegebenenfalls eine mechanische Energieeinbringung erfolgt oder
b) aus einer Blende mit wenigstens einer Eintrittsdüse und einer Prallplatte besteht, wobei im Zwischenraum zwischen der Blende und der Prallplatte gegebenenfalls eine mechanische Energieeinbringung erfolgt.

### Ausführungsform a)

Die Homogenisiervorrichtung zur Isolierung der Polyhydroxyalkanoate besteht beispielsweise aus einer Blende mit wenigstens einer Eintrittsdüse und einer Blende mit wenigstens einer Austrittsdüse, wobei die Düsen axial zueinander angeordnet sind. Im Zwischenraum zwischen den Blenden kann sich ein statischer Mischer befinden. Gegebenenfalls erfolgt in dem Zwischenraum zusätzlich eine mechanische Energieeinbringung.

Die nach dem erfindungsgemäßen Verfahren einsetzbaren Blenden weisen wenigstens eine Öffnung, d.h. wenigstens eine Düse auf. Dabei können die beiden Blenden jeweils eine beliebige Anzahl an Öffnungen aufweisen, bevorzugt jedoch nicht mehr als jeweils 5 Öffnungen, besonders bevorzugt nicht mehr als jeweils drei Öffnungen, ganz besonders bevorzugt nicht mehr als jeweils zwei Öffnungen und insbesondere bevorzugt nicht mehr als jeweils eine Öffnung. Beide Blenden können eine unterschiedliche oder die selbe Anzahl an Öffnungen aufweisen, bevorzugt haben beide Blenden die selbe Anzahl an Öffnungen. Im allgemeinen handelt es sich bei den Blenden um perforierte Platten mit mindestens je einer Öffnung.

In einer anderen Ausführungsform dieses erfindungsgemäßen Verfahrens ist die zweite Blende durch ein Sieb ersetzt, d.h. die zweite Blende hat eine Vielzahl von Öffnungen bzw. Düsen. Die einsetzbaren Siebe können einen großen Bereich an Porengrößen überspannen, in der Regel liegen die Porengrößen zwischen 0,1 und 250 µm, bevorzugt zwischen 0,2 und 200 µm, besonders bevorzugt zwischen 0,3 und 150 µm und insbesondere zwischen 0,5 und 100 µm.

Die Öffnungen bzw. Düsen können jede denkbare geometrische Form haben, sie können beispielsweise kreisrund, oval, eckig mit beliebige vielen Ecken, die gegebenenfalls auch abgerundet sein können, oder auch sternförmig sein. Bevorzugt haben die Öffnungen eine kreisrunde Form.

Die Öffnungen der Eintrittsblende haben in der Regeln einen Durchmesser von 0,05 mm bis 1 cm, bevorzugt von 0,08 mm bis 0,8 mm, besonders bevorzugt von 0,1 bis 0,5 mm und insbesondere von 0,2 bis 0,4 mm. Die Öffnungen der Austrittsblende haben in der Regeln einen Durchmesser von 0,5 mm bis 1 cm, bevorzugt von 5 mm bis 50 mm, besonders bevorzugt von 10 bis 20 mm.

Die beiden Blenden sind vorzugsweise so konstruiert, dass die Öffnungen bzw. Düsen axial zueinander angeordnet sind. Unter axialer Anordnung soll verstanden werden, dass die durch die Geometrie der Düsenöffnung erzeugte Strömungsrichtung bei beiden Blenden identisch ist. Die Öffnungsrichtungen der Eintritts- und Austrittsdüse müssen dazu nicht auf einer Linie liegen, sie können auch parallel verschoben sein, wie aus den obigen Ausführungen hervorgeht. Vorzugsweise sind die Blenden parallel ausgerichtet.

Es sind jedoch andere Geometrien, insbesondere nicht parallele Blenden oder unterschiedliche Öffnungsrichtungen der Ein- und Austrittsdüse möglich. Im Zweiblendensystem (Eintritts- und Austrittsblende) weist wie oben ausgeführt die Austrittsdüse größere Öffnungen auf. Dadurch kommt es zu einer Beruhigung der Turbulenz. Ein Prallblech ist in diesem Fall nicht notwendig.

Die Dicke der Blenden kann beliebig sein. Bevorzugt haben die Blenden eine Dicke im Bereich von 0,1 bis 100 mm, bevorzugt von 0,5 bis 30mm und besonders bevorzugt von 1 bis 10 mm. Dabei ist die Dicke (I) der Blenden so gewählt, dass der Quotient aus Durchmesser (d) der Öffnungen und Dicke (I) im Bereich von 1:1, bevorzugt 1:1,5 und besonders bevorzugt 1:2 beträgt.

Der Zwischenraum zwischen den beiden Blenden kann beliebig lang sein, in der Regel beträgt die Länge des Zwischenraums 1 bis 500 mm, bevorzugt 10 bis 300 mm und besonders bevorzugt 20 bis 100 mm.

Im Zwischenraum zwischen den Blenden kann sich ein statischer Mischer befinden, der die Strecke zwischen den beiden Blenden ganz oder teilweise ausfüllen kann. Bevorzugt erstreckt sich der statische Mischer über die gesamte Länge des Zwischenraums zwischen den beiden Blenden. Statische Mischer sind dem Fachmann bekannt. Es kann sich dabei beispielsweise um einen Ventil-Mischer handeln oder um einen statischen Mischer mit Bohrungen, einen aus geriffelten Lamellen oder einen aus ineinandergreifenden Stegen. Weiterhin kann es sich um einen statischen Mischer in Wendel-Form oder in N-Form oder um einen solchen mit heiz- oder kühlbaren Mischelementen handeln

Zusätzlich zu dem statischen Mischer kann in dem Zwischenraum zwischen den beiden Blenden noch eine mechanische Energieeinbringung erfolgen. Die Energie kann beispielsweise in Form von mechanischen Schwingungen, Ultraschall oder Rotationsenergie eingebracht werden. Dadurch wird eine turbulente Strömung erzeugt, die bewirkt, dass die Partikel im Zwischenraum nicht agglomerieren.

### Ausführungsform b)

Alternativ zu dieser ersten Variante kann die Mischvorrichtung aus einer Blende mit wenigstens einer Eintrittsdüse und einer Prallplatte bestehen, wobei sich im Zwischenraum zwischen der Blende und der Prallplatte gegebenenfalls ein statischer Mischer befindet. Alternativ oder zusätzlich zu dem statischen Mischer kann in dem Zwischenraum eine mechanische Energieeinbringung erfolgen.

Für die Blende mit Eintrittsdüse, dem Zwischenraum mit statischem Mischer und der mechanischen Energieeinbringung gilt das obengesagte.

In dieser Variante wird die zweite Blende durch eine Prallplatte ersetzt. Die Prallplatte hat in der Regel einen Durchmesser, der 0,5 bis 20 %, bevorzugt 1 bis 10 % kleiner ist als der Rohrdurchmesser an der Stelle, an der die Prallplatte eingebaut ist.

Generell kann die Prallplatte jede geometrische Form haben, bevorzugt in Form einer runden Scheibe, so dass in Frontalaufsicht ein Ringspalt zu sehen ist. Denkbar ist beispielsweise auch die Form eines Schlitzes oder eines Kanals.

Die Prallplatte kann analog zur zweiten Blende bei der oben beschriebenen Variante in unterschiedlichen Abständen zur ersten Blende angebracht sein. Dadurch ist der Zwischenraum zwischen der Blende und der Prallplatte beliebig lang, in der Regel beträgt die Länge des Zwischenraums 1 bis 500 mm, bevorzugt 10 bis 300 mm und besonders bevorzugt 20 bis 100 mm.

Das erfindungsgemäße Verfahren weist gegenüber denen aus dem Stand der Technik bekannten Verfahren einige Vorteile auf, da besonders hohe Ausbeuten des Polyhydroxyalkanoats mit hohem Molekulargewicht erhalten werden. Insbesondere lassen sich Polyhydroxyalkanoate mit Mn von 50.000 bis 2.000.000 und insbesondere von 100.000 bis 200.000 mit dieser Aufarbeitungsvariante realisieren.

Die Temperatur, bei der die Emulgierung der Rohemulsion zur feinteiligen Emulsion nach dem erfindungsgemäßen Verfahren erfolgt, beträgt in der Regel 0 bis 150°C, bevorzugt, 5 bis 80°C, besonders bevorzugt 20 bis 40°C. Dabei können alle in der Vorrichtung eingesetzten Homogenisiereinheiten temperierbar sein.

Die Homogenisierung wird in der Regel bei Drücken oberhalb des Atmosphärendrucks, d.h. > 1 bar durchgeführt. Dabei übersteigen die Drücke jedoch nicht einen Wert von 10 000 bar, so dass bevorzugt Homogenisierdrücke von > 1 bar bis 10 000 bar, bevorzugt 5 bis 2 500 bar und besonders bevorzugt von 100 bis 2000 bar eingestellt werden.

Die im erfindungsgemäßen Verfahren verwendeten Produktionszellkonzentrationen betragen etwa von 20 bis 300 g/L, bevorzugt 50-220 g/L.

Als Produktionszelle wird dabei jede Art von Zelle oder Zellverband bezeichnet; insbesondere solche Zellen tierischen, pflanzlichen oder mikrobiellen Ursprungs. Gleichfalls bevorzugt sind Produktionszellen rekombinanter Organismen. Besonders gut geeignete Produktionszellen sind Prokaryonten (einschliesslich der Archaea) oder Eukaryonten, besonders Bakterien einschliesslich Halobacterien und Methanococcen, Pilze, Insektenzellen, Pflanzenzellen und Säugerzellen, besonders bevorzugt Alcaligenes eutrophus, Escherichia coli, Bacillus subtilis, Bacillus. megaterium, Aspergillus oryzea, Aspergillus nidulans, Aspergillus niger, Pichia pastoris, Pseudomonas spec., Lactobacillen, Hansenula polymorpha, Trichoderma reesei, SF9 (bzw. verwandte Zellen). Besonders bevorzugt ist der Mikroorganismus Alcaligenes eutrophus.

Die Produktionszelle kann direkt nach der Kultivierung (z.B. Fermentation) in das erfindungsgemäße Verfahren eingesetzt werden; es ist aber auch möglich, die Produktionszelle erst abzutöten, beispielsweise durch Sterilisation, und ggf die Zellmasse durch Filtration des Kultivierungsmediums anzureichern.

Unter Polyhydroxyalkanoaten werden biotechnologisch hergestellte Polymere verstanden. Insbesondere sind darunter: Poly-3-hydroxybutyrat (P-3HB), Poly-3-hydroxybutyrat/co-3-hydroxyvaleriat (P-3HBco-3HV), Poly-3-hydroxybutyrat/co-4-hydroxybutyrat (P-3HB-co-4HB), Poly-3-hydroxybutyrat/co-3-hydroxyhexanoat (P-3HB-co-3HHx) und Poly-3-hydroxybutyrat/co-3-hydroxyoctanoat (P-3HB-co-3HO) zu verstehen.

### Verwendete Apparaturen:

Im Beispiel wurde als Hochdruckhomogenisator zum Aufschluss der Produktionszellen folgende Anordnung I gewählt. Als Eintrittsdüse wurde eine Blende mit 14 x 0,2 mm starken Bohrungen verwendet. Die Fermentationsbrühe lag als Suspension vor und wurde mit einem Druck von ca. 2000 atm durch die Blende gedrückt. Im Zwischenraum (15 mm lang und 8 mm im Durchmesser wurde die Suspension verwirbelt, bevor sie auf die zweite Blende auftrat, die als Austrittsdüse fungierte. Die Zellsuspension wurde durch eine konische Bohrung auf die Austrittsblende geführt und trat dann aus einer einzigen Bohrung (Durchmesser 1, 5 mm) aus dem Blendenblock aus. Die Austrittsblende war gegenüber den Bohrungen der Eintrittsdüse zentrisch angeordnet.

Als Düsenseparator wurde ein Gerät der Firma GEA Westfalia Typ HFC-15 verwendet.

### Beispiel 1 Isolierung von 3-Hydroxy-polyhydoxybutyrat (3-PHB) aus Alcaligenes eutrophus-Produktionszellen

i) Fermentation von 3-Hydroxypolyhydoxybutyrat in Alcaligenes eutrophus-Produktionszellen:
   Die Fermentation erfolgte nach Kim, Lee, Lee, Chang, Chang und Woo in Biotechnology and Bioengineering, Vol. 43, Seiten 892-898 (1994).
ii) Aufschluss der Zellen und Abtrennung der 3-PHB-Körnchen:
   3.300 Liter Alcaligenes eutrophus Fermentationsbrühe mit einem Gehalt von 90 g/l Biotrockenmasse, davon 80% PHB, wurden nach Vollendung der Fermentation auf 2°C im Fermenter abgefühlt. Die Brühe wird dann über einen Hochdruckhomogenisator I bei 2000 bar Druck gefahren. Da der Druck erst aufgebaut werden musste, wurden die ersten Liter Brühe getrennt gesammelt und in den Fermenter zurückgeführt. Die Fermentationsbrühe wurde vollständig über den Hochdruckhomogenisator gefahren.
   Die Wirksamkeit des Zellaufschlusses wurde durch Plattierung der Fermentationsbrühe vor und nach dem Aufschluss auf einem geeigneten Nähragar gemessen. Die Lebendzellzahl betrug vor Aufschluss 5 x 10¹⁰ cfu/ml (= 100%). Nach Zellaufschluss wurde die Lebendzellzahl in gleicher Weise bestimmt. Sie betrug 5 x 10⁶ cfu/ml. Dies entspricht einer Wirksamkeit des Hochdruckaufschlusses von 99,99%.
   Das Zellhomogenisat wurde dann über einen Düsenseparator der Firma GEA Westfalia Typ HFC-15 gefahren. Das Material, welches durch die zentrifugalen Kräfte abgeschieden wurde (Konzentrat) wurde von den Zelltrümmern, die nicht abgeschieden wurden (Überlauf) getrennt gesammelt. Gesamttrockensubstanz und PHB-Konzentration wurden jeweils bestimmt (Ergebnisse siehe Tabelle unten). Das Konzentrat wurde mit VE-Wasser auf das ursprüngliche Ausgangsvolumen verdünnt und erneut zentrifugiert. Der Vorgang wurde noch einmal wiederholt.
iii) Trocknung der 3-PHB-Körnchen
   Die aus ii) gewonnene 3-PHB-Suspension wurde in einem konventionellen Sprühtrockner sprühgetrocknet. Trocknungsgas war Stickstoff mit einer Gaseintrittstemperatur von 200°C, Gasaustrittstemperatur 90°C. Die PHB-Suspension wurde mit Hilfe einer Zweistoffdüse verdüst. Das trockene Produkt wurde mittels einer Zellenradschleuse aus dem Gasstrom ausgeschieden. Die Versuchsergebnisse sind in nachfolgender Tabelle aufgeführt.

| Fraktion | Gesamtmasse | Konzentration Trockensubstanz [g/kg] | Gesamtmasse Trockensubstanz [kg] | Konzentration 3-PHB [g/kg] | Gesamtmasse 3-PHB [kg] | Anteil 3-PHB [g/g] |
|---|---|---|---|---|---|---|
| Fermentationsbrühe | 3300 | 80 | 264 | 64 | 211 | 0,800 |
| Homogenisat | 3400 | 77,6 | 264 | 62,1 | 211 | 0,800 |
| 1. Konzentrat | 780 | 284,2 | 221,7 | 270 | 211 | 0,950 |
| 1. Überlauf | 2620 | 16,15 | 42,3 | 0 | 0 | 0,000 |
| 2. Konzentrat | 770 | 276,8 | 213,1 | 274 | 211 | 0,990 |
| 2. Überlauf | 2630 | 3,3 | 8,6 | 0 | 0 | 0,000 |
| 3. Konzentrat | 760 | 277,6 | 211 | 277,5 | 210,9 | 0,999 |
| 3. Überlauf | 2640 | 0,80 | 2,1 | 0 | 0 | 0,000 |
| Sprühtrockner | 215,2 | 980 | 210,9 | 980 | 210,9 | 0,999 |

## Patentansprüche

1. Verfahren zur Isolierung von Polyhydroxyalkanoaten aus Produktionszellen, **dadurch gekennzeichnet, dass**
i) die Produktionszellen aufgeschlossen werden und anschließend
ii) die Zellfragmente von den Polyhydroxyalkanoat-Körnchen mittels eines kontinuierlich arbeitenden Düsenseparators abgetrennt werden.

2. Verfahren nach Anspruch 1, **dadurch gekennzeichnet, dass** die Produktionszellen in Schritt i) mittels einer Hochdruckhomogenisiervorrichtung aufgeschlossen werden.

3. Verfahren nach Anspruch 2, **dadurch gekennzeichnet, dass** die Homogenisiervorrichtung
a) aus einer Blende mit wenigstens einer Eintrittsdüse und einer Blende mit wenigstens einer Austrittsdüse besteht, wobei im Zwischenraum zwischen den Blenden gegebenenfalls zusätzlich mechanische Energieeinbringung erfolgt oder
b) aus einer Blende mit wenigstens einer Eintrittsdüse und einer Prallplatte besteht, wobei im Zwischenraum zwischen der Blende und der Prallplatte gegebenenfalls mechanische Energieeinbringung erfolgt.

4. Verfahren nach Anspruch 1, **dadurch gekennzeichnet, dass** es sich bei der Produktionszelle um einen rekombinanten Organismus handelt.

5. Verfahren nach Anspruch 1, **dadurch gekennzeichnet, dass** es sich bei dem Polyhydroxyalkanoat um ein Poly-3-hydroxybutyrat (P-3HB), Poly-3-hydroxy-butyrat/co-3-hydroxyvaleriat (P-3HB-co-3HV), Poly-3-Hydroxybutyrat/co-4-hydroxybutyrat (P-3HB-co-4HB), Poly-3-hydroxybutyrat/co-3-hydroxyhexanoat (P-3HB-co-3HHx) oder Poly-3-hydroxybutyrat/co-3-hydroxyoctanoat (P-3HB-co-3HO) handelt.

6. Verfahren nach Anspruch 1, **dadurch gekennzeichnet, dass** die Produktionszelle vor dem Homogenisieren abgetötet wird.

## Claims

1. A method for isolating polyhydroxyalkanoates from production cells which comprises
i) disintegrating the production cells and subsequently
ii) separating off the cell fragments from the polyhydroxyalkanoate grains by means of a continuous jet separator.

2. The method according to claim 1, wherein the production cells are disintegrated in step i) by means of a high-pressure homogenizing device.

3. The method according to claim 2, wherein the homogenizing device
a) comprises an orifice plate having at least one inlet nozzle and an orifice plate having at least one outlet nozzle, in the intermediate space between the orifice plates, if appropriate, mechanical energy being additionally introduced or
b) comprises an orifice plate having at least one inlet nozzle and an impact plate, in the intermediate space between the orifice plate and the impact plate, if appropriate, mechanical energy being introduced.

4. The method according to claim 1, wherein the production cell is a recombinant organism.

5. The method according to claim 1, wherein the polyhydroxyalkanoate is a poly(3-hydroxybutyrate) (P-3HB), poly(3-hydroxybutyrate)/co-3-hydroxyvalerate (P-3HB-co-3HV), poly(3-hydroxybutyrate)/co-4-hydroxybutyrate (P-3HB-co-4HB), poly(3-hydroxybutyrate)/co-3-hydroxyhexanoate (P-3HB-co-3HHx) or poly(3-hydrroxybutyrate)/co-3-hydroxyoctanoate (P-3HB-co-3HO).

6. The method according to claim 1, wherein the production cell is killed before homogenization.

## Revendications

1. Procédé pour l'isolement de polyhydroxyalcanoates à partir de cellules de production, **caractérise en ce que**
i) les cellules de production sont décomposées, puis
ii) les fragments cellulaires sont séparés des granules de polyhydroxyalcanoate au moyen d'un séparateur à pulvérisateur fonctionnant en continu.

2. Procédé selon la revendication 1, **caractérise en ce que** les cellules de production sont décomposées dans l'étape i) au moyen d'un dispositif d'homogénéisation à haute pression.

3. Procédé selon la revendication 2, **caractérisé en ce que** le dispositif d'homogénéisation
a) est constitué par un diaphragme présentait au moins un pulvérisateur d'entrée et un diaphragme présentant au moins un pulvérisateur de sortie, une introduction d'énergie mélanique supplémentaire étant le cas échéant réalisée dans l'espace intermédiaire entre les diaphragmes ou
b) est constitué par un diaphragmé présentant au moins un pulvérisateur d'entrée et un déflecteur, une introduction d'énergie mélanique supplémentaire étant le cas échéant réalisée dans l'espace intermédiaire entre le diaphragme et le déflecteur.

4. Procédé selon la revendication 1, **caractérisé en ce qu'**il s'agit, pour la cellule de production, d'un organisme recombinant.

5. Procédé selon la revendication 1, **caractérisé en ce qu'**il s'agit, pour le polyhydroxyalcanoate, d'un poly-3-hydroxybutyrate (P-3HB), d'un poly-3-hydroxybutyrate/co-3-hydroxyvalériate (P-3HB-co-3HV), d'un poly-3-hydroxybutyratelco-4-hydroxybutyrate (P-3HB-co-4HB), d'un poly-3-hydroxybutyrate/co-3-hydroxyhexanoate (P-3HB-co-3HHx) ou d'un poly-3-hydroxybutyrate/co-3-hydroxyoctanoate (P-3HB-co-3HO).

6. Procédé selon la revendication 1, **caractérisé en ce que** les cellules de production sont tuées avant l'homogénéisation.
